**Europäisches Patentamt**

**European Patent Office**

**Office européen des brevets**

(19)

(11) Numéro de publication: **0 223 723**
**B1**

(12) **FASCICULE DE BREVET EUROPEEN**

(45) Date de publication du fascicule du brevet:
21.03.90

(51) Int. Cl.⁴: **C07C 65/03**, C07C 51/02

(21) Numéro de dépôt: 86420258.5

(22) Date de dépôt: 16.10.86

(54) **Procédé de séparation et de purification de l'acide parahydroxybenzoïque.**

(30) Priorité: 29.10.85 FR 8516260

(43) Date de publication de la demande:
27.05.87 Bulletin 87/22

(45) Mention de la délivrance du brevet:
21.03.90 Bulletin 90/12

(84) Etats contractants désignés:
AT BE CH DE ES GB GR IT LI LU NL SE

(56) Documents cités:
FR-A- 1 061 806
FR-A- 1 539 528

(73) Titulaire: RHONE-POULENC CHIMIE, 25, quai Paul
Doumer, F-92408 Courbevole Cédex(FR)

(72) Inventeur: Cocco, Roger, Rue de la Croix Rouge Solaize,
F-69360 Saint-Symphorien d'Ozon(FR)

(74) Mandataire: Vignally, Noel et al, RHONE-POULENC
INTERSERVICES Service Brevets Chimie Centre de
Recherches de Saint-Fons B.P. 62, F-69192 Saint-Fons
Cedex(FR)

ACTORUM AG

## Description

La présente invention concerne la séparation et la purification de l'acide parahydroxybenzoïque et, plus particulièrement, la précipitation de l'acide parahydroxybenzoïque à partir de son bisel et/ou de son monosel de potassium et sa purification.

Le bisel et/ou le monosel de potassium de l'acide parahydroxybenzoïque peut être préparé notamment par carboxylation du phénate de potassium par de l'anhydride carbonique. Généralement la réaction est conduite sous quelques bars de pression (2 à 10 bars) et à une température de l'ordre de 200°C.

Fréquemment le phénate est carboxylé en présence de phénol libre et la réaction est opérée dans un milieu liquide dispersant. On peut pour cette préparation se référer par exemple au brevet français No. 1 564 997 ou encore au brevet français No. 2 065 098.

En fin de réaction la masse réactionnelle est traitée afin d'en séparer les sels de potassium des acides hydroxybenzoïques et hydroxyphtaliques obtenus, en l'occurrence essentiellement le bisel et/ou le monosel potassique de l'acide parahydroxybenzoïque et dans des proportions nettement moindres le salicylate de potassium et l'hydroxy-4 isophtalate de potassium.

Ce traitement peut consister notamment en une addition d'eau, qui dissout les sels de potassium des acides hydroxybenzoïques et hydroxyphtaliques, et en une extraction liquide-liquide à l'aide d'un solvant non miscible à l'eau. Une telle opération conduit ainsi à une solution organique contenant en particulier le phénol libre et une solution aqueuse contenant les sels de potassium indiqués précédemment.

Le procédé selon l'invention a pour but d'apporter un perfectionnement à la précipitation et à la purification de l'acide parahydroxybenzoïque à partir de telles solutions aqueuses.

En effet, généralement le traitement des solutions aqueuses de bisel et/ou de monosel de potassium de l'acide parahydroxybenzoïque consiste à précipiter l'acide parahydroxybenzoïque et les autres acides organiques par un acide minéral fort et notamment par de l'acide sulfurique. Ce traitement nécessite un fort excès d'acide minéral fort et plusieurs lavages successifs à l'eau pour éliminer le maximum de sel minéral formé (sulfate de potassium le plus souvent). Ensuite l'acide parahydroxybenzoïque obtenu peut être cristallisé une ou plusieurs fois pour diminuer le taux des autres acides organiques qu'il contient.

L'acide parahydroxybenzoïque obtenu par le procédé habituel contient des quantités de sulfate de potassium, et à un moindre degré d'acide salicylique et d'acide hydroxy-4 isophtalique, qui sont encore excessives pour certaines applications alimentaires de l'acide parahydroxybenzoïque.

La présente invention se propose de pallier ces inconvénients. En effet, elle permet selon les besoins soit d'obtenir plus simplement un acide parahydroxybenzoïque de pureté suffisante pour des usages courants, soit d'obtenir un acide parahydroxybenzoïque de très grande pureté, avec également une mise en oeuvre plus simple que dans les procédés antérieurs.

Elle consiste en un procédé de précipitation et de purification de l'acide parahydroxybenzoïque à partir d'une solution aqueuse de bisel et/ou de monosel de potassium de l'acide parahydroxybenzoïque, caractérisé par la succession suivante de phases :

1. - addition à ladite solution aqueuse d'un solvant organique de l'acide parahydroxybenzoïque, en quantité suffisante pour dissoudre l'acide parahydroxybenzoïque correspondant aux sels de potassium engagés ;

2. - addition d'un acide minéral fort en quantité au moins égale à la stoechiométrie par rapport au bisel et/ou monosel de potassium de l'acide parahydroxybenzoïque ;

3. - séparation d'une phase essentiellement organique contenant l'acide parahydroxybenzoïque et d'une phase aqueuse contenant le sel minéral de potassium formé.

Comme acide minéral fort, on peut utiliser tout acide capable de déplacer l'acide parahydroxybenzoïque de son bisel et/ou de son monosel de potassium; on peut citer à titre d'exemples non limitatifs l'acide sulfurique, l'acide chlorhydrique et l'acide phosphorique. Pour des questions de prix ou de moindre corrosion, on préfère généralement utiliser l'acide sulfurique.

Dans la description du procédé de l'invention, on se référera donc par commodité, le plus souvent à l'acidification par l'acide sulfurique, mais sans que soit pour autant exclu l'empoi d'autres acides minéraux forts.

Les solvants organiques qui conviennent pour la mise en oeuvre du procédé selon l'invention sont les solvants dans lesquels l'acide parahydroxybenzoïque est soluble tandis que le sulfate de potassium n'est pas soluble ou très peu soluble.

Ils peuvent être choisis parmi les éthers aliphatiques, les éthers aliphatiques substitués, les cétones aliphatiques, les cétones aliphatiques halogénées, les aldéhydes aliphatiques et les alcools aliphatiques.

Pour des questions pratiques et économiques, il n'est pas souhaitable d'utiliser des solvants organiques ayant un point d'ébullition très élevé, car lors de l'isolement de l'acide parahydroxybenzoïque le solvant sera généralement éliminé par distillation et l'acide parahydroxybenzoïque devra contenir en fin de purification le minimum possible de traces du solvant employé.

C'est ainsi que l'on préférera un solvant ayant un point d'ébullition inférieur ou égal à 120°C et, plus particulièrement encore, inférieur ou égal à 100°C.

A titre d'exemples de ces solvants on peut citer:
– des éthers aliphatiques tels que l'oxyde de diisopropyle; l'oxyde de méthyle et de tertiobutyle; l'oxyde d'éthyle et d'isopropyle; l'oxyde d'éthyle et de propyle; l'oxyde de butyle et d'éthyle; l'oxyde d'éthyle et d'isobutyle; l'oxyde d'éthyle et de tertiobutyle; l'oxyde de butyle et de méthyle; l'oxyde d'isobutyle et de méthyle; l'oxyde de méthyle et de pentyle; l'oxyde de diéthyle; l'oxyde de dipropyle; l'oxyde d'isopropyle et de propyle; l'oxyde d'éthyle et de propynyle-1; l'oxyde d'éthyle et de propynyle-2; l'oxyde d'éthyny- le et de propyle; l'oxyde d'allyle et d'éthyle; l'oxyde d'allyle et d'isopropyle; l'oxyde d'isopropyle et de vinyle ou l'oxyde d'isobutyle et de vinyle;
– des éthers aliphatiques halogénés tels que l'oxyde de bromo-2 éthyle et d'éthyle; l'oxyde de chloro-2 éthyle et d'éthyle;
– des cétones aliphatiques telles que l'acétone; la butanone-2; la méthyl-3 butanone-2; la diméthyl-3,3 butanone-2; la pentanone-2 ou la pentanone-3;
– des cétones aliphatiques chlorées telles que la chloro-3 butanone-2 ou la chloro-1 propanone-2;
– des aldéhydes aliphatiques tels que le propanal; le butanal; le méthyl-2 butanal; le méthyl-3 butanal; le pentanal; le méthoxyéthanal ou l'éthoxyéthanal;
– des aldéhydes aliphatiques chlorés tels que le chloroéthanal; le dichloroéthanal; le chloro-2 propanal ou le chloro-2 méthyl-2 propanal;
– des alcools aliphatiques tels que le méthanol; l'éthanol; l'isopropanol; le n-propanol, le n-butanol; l'isobutanol ou le tertiobutanol.

Parmi les solvants qui sont utilisés dans le procédé selon l'invention, on préfère plus particulièrement les éthers aliphatiques, les éthers aliphatiques chlorés, les cétones aliphatiques et les cétones aliphatiques chlorées.

Parmi ces solvants préférés, on choisit encore plus préférentiellement les éthers aliphatiques, surtout ceux qui ne sont pas miscibles ou qui sont peu miscibles à l'eau.

La quantité de solvant organique utilisée peut varier très largement.

La limite inférieure est constituée par la solubilité de l'acide parahydroxybenzoïque dans ledit solvant à la température à laquelle on opère.

La limite supérieure est généralement non critique, mais il est bien évident qu'il n'est pas intéressant au plan économique d'opérer à de trop faibles concentrations finales d'acide parahydroxybenzoïque dans le solvant.

En outre lorsque le solvant organique utilisé est miscible à l'eau, il est nécessaire que la concentration finale d'acide parahydroxybenzoïque dans ledit solvant soit suffisante, pour qu'il se produise une démixtion entre une couche aqueuse contenant le sel minéral de potassium formé et une couche essentiellement organique contenant l'acide parahydroxybenzoïque.

Pour ces raisons, la quantité de solvant utilisée est telle que la concentration finale en acide parahydroxybenzoïque dans ledit solvant est généralement au moins égale à 8 % en poids.

La température à laquelle est mis en oeuvre le procédé selon l'invention est peu critique. Elle se situe habituellement entre 10°C et la température d'ébullition du solvant organique utilisé.

Cependant dans la mesure du possible, il vaut mieux éviter des températures trop basses qui peuvent conduire à une cristallisation du sel minéral de potassium formé et notamment du sulfate de potassium ou des températures trop élevées auxquelles la solubilité de l'acide parahydroxybenzoïque dans l'eau est augmentée.

C'est pourquoi on opère de préférence à des températures de 40°C à 80°C, sans que cela représente un impératif.

La quantité d'acide minéral fort et plus particulièrement d'acide sulfurique utilisée est généralement légèrement supérieure à la quantité théoriquement nécessaire pour déplacer l'acide parahydroxybenzoïque et les acides salicylique et hydroxy-4 isophtalique de leurs sels respectifs de potassium.

Cet excès, qui est souvent d'environ de 5% à 10% de la quantité stoechiométrique, permet une fin de réaction plus rapide.

L'acide sulfurique est introduit habituellement sous forme d'une solution aqueuse telle que celles qui sont disponibles dans le commerce ; le plus fréquemment on utilise des solutions aqueuses ayant une concentration de 60% à 98% en poids.

En pratique, le procédé selon la présente invention peut être réalisé de la manière suivante :

A la solution aqueuse, contenant le bisel et/ou le monosel de potassium de l'acide parahydroxybenzoïque à une concentration de 20 à 45% en poids et, le cas échéant, les sels de potassium des autres acides, est ajouté un solvant organique tel que défini précédemment en quantité suffisante pour dissoudre l'acide parahydroxybenzoïque correspondant aux sels de potassium engagés.

Selon le cas on obtient une phase homogène ou deux phases liquides, ce dernier cas représentant une variante préférentielle du présent procédé.

On ajoute alors sous agitation l'acide sulfurique en quantité représentant environ 105% à 110% de la théorie nécessaire (la théorie étant 1 mole de $H_2SO_4$ pur pour 1 mole de bisel de potassium de l'acide parahydroxybenzoïque ou de l'acide salicylique, 0,5 mole de $H_2SO_4$ pour 1 mole de monosel de potassium de l'acide parahydroxybenzoïque ou de l'acide salicylique et 1 mole de $H_2SO_4$ pour 1 mole d'hydroxy-4 isophtalate de potassium).

On amène la température à la valeur désirée.

Lorsque la réaction est terminée, on obtient deux phases liquides :
– une phase aqueuse contenant pratiquement tout le sulfate de potassium formé ;
– une phase essentiellement organique contenant la quasi-totalité de l'acide parahydroxybenzoïque formé.

En effet :
– soit, et cela représente la variante préférée, on a mis en oeuvre un solvant non miscible à l'eau et qui par conséquent décante ;
– soit l'on a utilisé un solvant miscible à l'eau et l'on observe une démixtion entre la phase aqueuse chargée de sulfate de potassium et la phase organique chargée d'acide parahydroxybenzoïque.

On opère ensuite la séparation de ces deux phases par décantation. La phase aqueuse contient généralement plus de 10 % en poids de sulfate de potassium (qui peut, si on le souhaite, être récupéré) et généralement moins de 1 % en poids d'acide parahydroxybenzoïque.

La phase essentiellement organique est alors traitée de manière connue en soi pour séparer l'acide parahydroxybenzoïque.

On peut par exemple, si les applications envisagées ne nécessitent pas une pureté très supérieure à celle obtenue pour les procédés courants de séparation de l'acide parahydroxybenzoïque, procéder à une atomisation de ladite phase organique, c'est-à-dire à sa pulvérisation à travers une buse à une température permettant la vaporisation instantanée du solvant. Ce procédé est simple et conduit à un acide parahydroxybenzoïque de pureté au moins comparable à celle que donnent les procédés antérieurs.

On peut également, et c'est généralement la variante préférée, procéder à une distillation du solvant organique, suivie d'un refroidissement et d'une cristallisation de l'acide parahydroxybenzoïque par addition d'eau.

L'acide parahydroxybenzoïque obtenu selon cette dernière variante présente un taux de sulfate de potassium très faible, généralement inférieur à 0,002% en poids et un taux d'acide salicylique et d'acide hydroxy-4 isophtalique inférieur à 0,02%.

Un tel acide parahydroxybenzoïque peut être utilisé dans les applications alimentaires les plus exigeantes.

En outre on note que les cristaux d'acide parahydroxybenzoïque ainsi préparés ont une granulométrie qui peut être contrôlée lors de la cristallisation en agissant sur la température à laquelle cette opération est effectuée. On obtient des aiguilles plus épaisses et une densité apparente plus élevée, ce qui permet une nette amélioration de la coulabilité de l'acide parahydroxybenzoïque.

Le procédé de l'invention peut être mis en oeuvre de manière discontinue ou de manière continue. Il peut facilement être réalisé dans le cadre des procédés habituels de préparation d'acide parahydroxybenzoïque à partir du phénate de potassium.

L'exemple qui suit illustre la présente invention.

## EXEMPLE

Dans un réacteur en verre de 2000 cm3, équipé d'une agitation efficace, on charge :
– 1445 g d'une solution aqueuse à 16 % en poids de bisel potassique de l'acide parahydroxybenzoïque, contenant 1% en mole de salicylate de potassium par rapport au bisel de l'acide parahydroxybenzoïque;
– 743 g d'oxyde de méthyle et de tertiobutyle.

On agite et on rajoute 166 g d'une solution aqueuse d'acide sulfurique à 71% en poids (1,20 mole de $H_2SO_4$) : le pH de la phase aqueuse est alors voisin de 2.

On poursuit l'agitation pendant 30 minutes tout en amenant la température à 40°C.

On laisse ensuite décanter pendant 30 minutes à environ 40°C ; on obtient deux phases :
– une phase aqueuse inférieure,
– une phase organique supérieure.

On soutire la phase aqueuse toujours vers 40°C.

La phase organique contient :
– 705 g d'oxyde de méthyle et de tertiobutyle ;
– 42 g d'eau ;
– 126 g d'acide parahydroxybenzoïque ;
– 1,4 g d'acide salicylique ;
– 0,01 g de sulfate de potassium.

La phase organique est traitée de la manière suivante :
– on distille l'oxyde de méthyle et de tertiobutyle tout en maintenant le volume constant de la phase par addition progressive d'eau ;
– on arrête la distillation lorsque la température du condenseur est à 98–100°C.

On obtient ainsi une nouvelle phase aqueuse comportant des cristaux d'acide parahydroxybenzoïque en suspension.

On refroidit cette suspension de 100 à 85°C en deux heures, puis de 85°C à 40°C en une heure.

On filtre à 40°C l'acide parahydroxybenzoïque qui a cristallisé. On lave par environ 150 g d'eau l'acide parahydroxybenzoïque, puis on le sèche.

On obtient 120 g d'acide parahydroxybenzoïque sous forme d'un solide blanc cristallisé, ayant une bonne coulabilité.

L'acide parahydroxybenzoïque contient comme impuretés :
– moins de 0,001% de sulfate de potassium ;
– 0,012% d'acide salicylique.

Le rendement de l'isolement et de la purification de l'acide parahydroxybenzoïque par rapport à son bisel de potassium engagé est d'environ 80%. Il peut être amélioré notamment en diminuant les quantités d'eau utilisées.

## Revendications

1. Procédé de purification de l'acide parahydroxybenzoïque à partir d'une solution aqueuse de bisel et/ou de monosel de potassium de l'acide parahydroxybenzoïque, caractérisé par la succession suivante de phases:

(1) addition à ladite solution aqueuse d'un solvant organique de l'acide parahydroxybenzoïque, en quantité pour dissoudre l'acide parahydroxybenzoïque correspondant aux sels de potassium engagés;
(2) addition d'acide minéral fort en quantité au moins égale à la stoechiométrie par rapport au bisel et/ou au monosel de potassium de l'acide parahydroxybenzoïque;
(3) séparation d'une phase essentiellement organique contenant l'acide parahydroxybenzoïque et d'une phase aqueuse contenant le sel minéral de potassium formé, et son traitement de manière connue en soi pour séparer l'acide parahydroxybenzoïque;

2. Procédé selon la revendication 1, caractérisé en ce que l'acide minéral fort utilisé est l'acide sulfurique.

3. Procédé selon l'une des revendications 1 ou 2, caractérisé en ce que le solvant organique utilisé est choisi parmi les éthers aliphatiques, les éthers aliphatiques substitués, les cétones aliphatiques, les cétones aliphatiques halogénées, les aldéhydes aliphatiques et les alcools aliphatiques.

4. Procédé selon l'une des revendications 1 à 3, caractérisé en ce que le solvant organique est choisi parmi les éthers aliphatiques, les éthers aliphatiques chlorés, les cétones aliphatiques et les cétones aliphatiques chlorées.

5. Procédé selon l'une des revendications 1 à 4, caractérisé en ce que le solvant organique possède un point ébullition inférieur ou égal à 120°C et de préférence inférieur ou égal à 100°C.

6. Procédé selon l'une des revendications 1 à 5, caractérisé en ce que le solvant organique est choisi parmi:
- des éthers aliphatiques tels que l'oxyde de diisopropyle; l'oxyde de méthyle et de tertiobutyle; l'oxyde d'éthyle et d'isopropyle; l'oxyde d'éthyle et de propyle; l'oxyde de butyle et d'éthyle; l'oxyde d'éthyle et d'isobutyle; l'oxyde d'éthyle et de tertiobutyle; l'oxyde de butyle et de méthyle; l'oxyde d'isobutyle et de méthyle; l'oxyde de méthyle et de pentyle; l'oxyde de diéthyle; l'oxyde de dipropyle; l'oxyde d'isopropyle et de propyle; l'oxyde d'éthyle et de propynyle-1; l'oxyde d'éthyle et de propynyle-2; l'oxyde d'éthynyle et de propyle; l'oxyde d'allyle et d'éthyle; l'oxyde d'allyle et d'isopropyle; l'oxyde d'isopropyle et de vinyle ou l'oxyde d'isobutyle et de vinyle;
- des éthers aliphatiques halogénés tels que l'oxyde de bromo-2 éthyle et d'éthyle; l'oxyde de chloro-2 éthyle et d'éthyle;
- des cétones aliphatiques telles que l'acétone; la butanone-2; la méthyl-3 butanone-2; la diméthyl-3,3 butanone-2; la pentanone-2 ou la pentanone-3;
- des cétones aliphatiques chlorées telles que la chloro-3 butanone-2 ou la chloro-1 propanone-2.

7. Procédé selon l'une des revendications 1 à 6, caractérisé en ce que le solvant organique est choisi parmi les éthers aliphatiques non miscibles ou peu miscibles à l'eau.

8. Procédé selon l'une des revendications 1 à 7, caractérisé en ce que la quantité de solvant organique est telle que la concentration finale en acide parahydroxybenzoïque dans ledit solvant soit au moins égale à 8% en poids.

9. Procédé selon l'une des revendications 1 à 8, caractérisé en ce que la température à laquelle il est mis en œuvre est comprise entre 10°C et la température d'ébullition du solvant organique et de préférence entre 40°C et 80°C.

10. Procédé selon l'une revendications 1 à 9, caractérisé en ce que l'on utilise environ 105% à 110% de la quantité stoechiométrique d'acide sulfurique.

**Patentansprüche**

1. Verfahren zur Reinigung der Parahydroxybenzoesäure aus einer wäßrigen Lösung des Kaliumbisalzes und/oder -monosalzes der Parahydroxybenzoesäure, gekennzeichnet durch die folgende Aufeinanderfolge der Phasen:

(1) Zugabe eines organischen Lösungsmittels für Parahydroxybenzoesäure zu dieser wäßrigen Lösung in ausreichender Menge, um die Parahydroxybenzoesäure entsprechend den eingesetzten Kaliumsalzen zu lösen;
(2) Zugabe einer starken Mineralsäure in einer Menge mindestens gleich der Stöchiometrie in bezug auf das Kaliumbisalz und/oder -monosalz der Parahydroxybenzoesäure;
(3) Abtrennung einer im wesentlichen organischen Phase, enthaltend die Parahydroxybenzoesäure, und einer wäßrigen Phase, enthaltend das gebildete Kaliummineralsalz, und Behandlung in an sich bekannter Weise zur Abtrennung der Parahydroxybenzoesäure.

2. Verfahren gemäß Anspruch 1, dadurch gekennzeichnet, daß die verwendete starke Mineralsäure Schwefelsäure ist.

3. Verfahren gemäß einem der Ansprüche 1 oder 2, dadurch gekennzeichnet, daß das verwendete organische Lösungsmittel ausgewählt ist unter den aliphatischen Ethern, den substituierten, aliphatischen Ethern, den aliphatischen Ketonen, den halogenierten, aliphatischen Ketonen, den aliphatischen Aldehyden und den aliphatischen Alkoholen.

4. Verfahren gemäß einem der Ansprüche 1 bis 3, dadurch gekennzeichnet, daß das organische Lösungsmittel ausgewählt ist unter den aliphatischen Ethern, den chlorierten, aliphatischen Ethern, den aliphatischen Ketonen und den chlorierten, aliphatischen Ketonen.

5. Verfahren gemäß einem der Ansprüche 1 bis 4, dadurch gekennzeichnet, daß das organische Lösungsmittel einen Siedepunkt unterhalb oder gleich 120°C und vorzugsweise unterhalb oder gleich 100°C besitzt.

6. Verfahren gemäß einem der Ansprüche 1 bis 5, dadurch gekennzeichnet, daß das organische Lösungsmittel ausgewählt ist unter:
den aliphatischen Ethern, wie Diisopropylether, Methyl-tert.-butylether, Ethylisopropylether, Ethylpropylether, Butylethylether, Ethylisobutylether, Ethyl-tert.-butylether, Butylmethylether, Isobutylmethylether, Methylpentylether, Diethylether, Dipropylether, Isopropylpropylether, Ethyl-1-propinylether, Ethyl-2-propinylether, Ethinylpropylether, Allylethylether, Allylisopropylether, Isopropylvinylether oder Isobutylvinylether;
den halogenierten, aliphatischen Ethern, wie 2-Bromethylethylether, 2-Chlorethylethylether;
den aliphatischen Ketonen, wie Aceton, 2-Butanon, 3-Methyl-2-butanon, 3,3-Dimethyl-2-butanon, 2-Pentanon oder 3-Pentanon;
den chlorierten, aliphatischen Ketonen, wie 3-Chlor-2-butanon oder 1-Chlor-2-propanon.

7. Verfahren gemäß einem der Ansprüche 1 bis 6, dadurch gekennzeichnet, daß das organische Lösungsmittel ausgewählt ist unter den aliphatischen, mit Wasser nicht oder wenig mischbaren Ethern.

8. Verfahren gemäß einem der Ansprüche 1 bis 7, dadurch gekennzeichnet, daß die Menge an organischem Lösungsmittel derart ist, daß die Endkonzentration an Parahydroxybenzoesäure in diesem Lösungsmittel mindestens gleich 8 Gew.-% ist.

9. Verfahren gemäß einem der Ansprüche 1 bis 8, dadurch gekennzeichnet, daß die Temperatur, bei der es durchgeführt wird, zwischen 10°C und der Siedetemperatur des organischen Lösungsmittels und vorzugsweise zwischen 40°C und 80°C liegt.

10. Verfahren gemäß einem der Ansprüche 1 bis 9, dadurch gekennzeichnet, daß man etwa 105% bis 110% der stöchiometrischen Menge an Schwefelsäure verwendet.

## Claims

1. Process for the purification of para-hydroxybenzoic acid from an aqueous solution of the dipotassium and/or monopotassium salt of para-hydroxybenzoic acid, characterized by the following sequence of stages:

(1) addition to the said solution of an organic solvent for para-hydroxybenzoic acid, in a quantity sufficient to dissolve the para-hydroxybenzoic acid corresponding to the potassium salts employed;

(2) addition of a strong inorganic acid in a quantity at least equal to the stoichiometric amount relative to the dipotassium and/or monopotassium salt of para-hydroxybenzoic acid;

(3) separation of an essentially organic phase containing para-hydroxybenzoic acid and of an aqueous phase containing the inorganic potassium salt formed and its treatment in a manner known per se to separate the para-hydroxybenzoic acid.

2. Process according to claim 1, characterized in that the strong inorganic acid used is sulphuric acid.

3. Process according to one of claims 1 or 2, characterized in that the organic solvent used is chosen from aliphatic ethers, substituted aliphatic ethers, aliphatic ketones, halogenated aliphatic ketones, aliphatic aldehydes and aliphatic alcohols.

4. Process according to one of claims 1 to 3, characterized in that the organic solvent is chosen from aliphatic ethers, chlorinated aliphatic ethers, aliphatic ketones and chlorinated aliphatic ketones.

5. Process according to one of claims 1 to 4, characterized in that the organic solvent has a boiling point less than or equal to 120°C and preferably less than or equal to 100°C.

6. Process according to one of claims 1 to 5, characterized in that the organic solvent is chosen from: aliphatic ethers such as diisopropyl ether; methyl tert-butyl ether; ethyl isopropyl ether; ethyl propyl ether; butyl ethyl ether; ethyl isobutyl ether; ethyl tert-butyl ether; butyl methyl ether; isobutyl methyl ether; methyl pentyl ether; diethyl ether; dipropyl ether; isopropyl propyl ether; ethyl 1-propynyl ether; ethyl 2-propynyl ether; ethynyl propyl ether; allyl ethyl ether; allyl isopropyl ether; isopropyl vinyl ether or isobutyl vinyl ether;
halogenated aliphatic ethers such as 2-bromoethyl ethyl ether; 2-chloroethyl ethyl ether;
aliphatic ketones such as acetone; 2-butanone; 3-methyl-2-butanone; 3,3-dimethyl-2-butanone; 2-pentanone or 3-pentanone;
chlorinated aliphatic ketones such as 3-chloro-2-butanone or 1-chloro-2-propanone.

7. Process according to one of claims 1 to 6, characterized in that the organic solvent is chosen from aliphatic ethers immiscible or only slightly miscible with water.

8. Process according to one of claims 1 to 7, characterized in that the quantity of organic solvent is such that the final para-hydroxybenzoic acid concentration in the said solvent is at least equal to 8% by weight.

9. Process according to one of claims 1 to 8, characterized in that the temperature at which it is carried out is between 10°C and the boiling point of the organic solvent and preferably between 40°C and 80°C.

10. Process according to one of claims 1 to 9, characterized in that approximately 105% to 110% of the stoichiometric quantity of sulphuric acid is used.